# EUROPÄISCHE PATENTANMELDUNG

(11) **EP 2 011 808 A1**
(43) Veröffentlichungstag der Anmeldung: **07.01.2009**
(21) Anmeldenummer: 07021764.1
(22) Anmeldetag: 09.11.2007
(51) Int. Cl.: C08G 18/10, C08G 18/48, C09J 175/12

(54) **Medizinische Klebstoffe für die Chirurgie**

(30) Priorität: 03.07.2007 EP 07012984
(71) Anmelder: Bayer MaterialScience AG, 51368 Leverkusen (DE)
(72) Erfinder: Heckroth, Heike, Dr., 51519 Odenthal (DE); Köhler, Burkhard, Dr., 34289 Zierenberg (DE); Dörr, Sebastian, Dr., 40597 Düsseldorf (DE)

(57) **Zusammenfassung**

Die vorliegende Erfindung betrifft neuartige, schnell härtende Klebstoffe auf Basis hydrophiler Polyisocyanat-Prepolymere für den Einsatz in der Chirurgie.

## Beschreibung

Die vorliegende Erfindung betrifft neuartige, schnell härtende Klebstoffe auf Basis hydrophiler Polyisocyanat-Prepolymere für den Einsatz in der Chirurgie.

In den vergangenen Jahren hat sich ein steigendes Interesse entwickelt, chirurgische Nähte durch den Einsatz von geeigneten Klebstoffen zu ersetzen oder zu unterstützen. Besonders im Bereich der plastischen Chirurgie, in der auf dünne, möglichst unsichtbare Narben Wert gelegt wird, werden vermehrt Klebstoffe eingesetzt.

Gewebekleber müssen eine Reihe von Eigenschaften haben, um bei den Chirurgen als Nahtersatz akzeptiert zu werden. Hierzu gehören eine leichte Verarbeitbarkeit und eine Eingangsviskosität, so dass der Klebstoff nicht in tiefere Gewebeschichten eindringen oder verlaufen kann. In der klassischen Chirurgie wird eine schnelle Aushärtung erfordert, wogegen in der plastischen Chirurgie eine Korrektur der Klebenaht möglich sein sollte und damit die Aushärtungsgeschwindigkeit nicht zu schnell sein darf (ca. 5 min). Die Klebeschicht sollte ein flexibler, transparenter Film sein, der nicht in einem Zeitraum kleiner drei Wochen abgebaut wird. Der Klebstoff muß biokompatibel sein und darf weder Histotoxicität noch Thrombogenität oder ein allergenes Potential besitzen.

Diverse Materialien, die als Gewebekleber eingesetzt werden, sind im Handel erhältlich. Hierzu gehören die Cyanacrylate Dermabond® (Octyl-2-Cyanoacrylat) und Histoacryl Blue® (Butyl-Cyanoacrylat). Die schnelle Aushärtezeit, sowie die Sprödigkeit der Klebestelle limitieren jedoch den Einsatz. Durch die schlechte Bioabbaubarkeit sind Cyanacrylate nur für äußerliche chirurgische Nähte geeignet.

Als Alternative zu den Cyanacrylaten stehen biologische Klebstoffe wie peptidbasierte Substanzen (BioGlue®) oder Fibrinkleber (Tissucol) zur Verfügung. Neben den hohen Kosten zeichnen sich Fibrinkleber durch eine relative schwache Klebstärke und einen schnellen Abbau aus, so dass dieser nur bei kleineren Schnitten auf nicht gespannter Haut anwendbar ist.

Isocyanat-haltige Klebstoffe basieren alle auf einem aromatischen Diisocyanat und einem hydrophilen Polyol, wobei bevorzugt die Isocyanate TDI und MDI eingesetzt werden (US 20030135238, US 20050129733). Beide können, um die Reaktivität zu erhöhen, Elektronen ziehende Substituenten tragen (WO-A 03/9323).

Schwierigkeiten waren bislang die geringe mechanische Stärke (US 5,156,613), zu langsame Aushärtungsgeschwindigkeit (US 4,806,614), die zu schnelle Bioabbaubarkeit (US 6,123,667) sowie unkontrollierte Schwellung (US 6,265,016).

Gemäß Patent US 20030135238 stellen ausschließlich Polyurethan-Prepolymere mit trifunktioneller oder verzweigter Struktur, die dazu in der Lage sind, Hydrogele zu bilden, geeignete Klebstoffe dar. Der Klebstoff muß dabei in der Lage sein, eine kovalente Bindung zum Gewebe auszubilden. US 20030135238 und US 20050129733 beschreiben' die Synthese trifunktioneller, Ethylenoxid reichet TDI-und IPDI- (US 20030135238) basierter Prepolymere, die mit Wasser oder mit Gewebeflüssigkeiten zum Hydrogel reagieren. Ausreichend schnelle Aushärtung wurde bislang nur bei Verwendung aromatischer Isocyanate erreicht, welche jedoch unter Schaumbildung reagieren. Dadurch kommt es zu einem Eindringen des Klebstoffes in die Wunde und damit zum Auseinanderdrücken der Wundränder, was eine schlechtere Heilung mit verstärkter Narbenbildung mit sich bringt. Durch die Schaumbildung ist zudem die mechanische Festigkeit sowie die Haftung der Klebeschicht herabgesetzt. Aufgrund der hohen Reaktivität der Prepolymere kommt es zudem zu einer Reaktion der Isocyanatreste mit dem Gewebe, wobei oftmals eine Denaturierung, erkennbar durch Weißfärbung des Gewebes, auftritt.

Als Ersatz der aromatischen Isocyanate wurde Lysindiisocyanat untersucht, welches jedoch aufgrund seiner geringen Reaktivität nicht oder nur langsam mit Gewebe reagiert (US 20030135238).

Aliphatische Isocyanate wurden, um die Reaktivität zu erhöhen, fluoriert (US 5,173,301), wodurch es jedoch spontan zu einer Selbstpolymerisation des Isocyanates kam.

EP-A 0 482 467 beschreibt die Synthese eines chirurgischen Klebstoffes basierend auf einem aliphatischen Isocyanat (bevorzugt HDI) und einem Polyethylenglycol (Carbowax 400). Die Aushärtung erfolgt bei Zugabe von 80-100 % Wasser und einem Metallcarboxylat (Kaliumoctoat) als Katalysator wobei sich ein Schaum bildet, der mit Silikonöl stabilisiert wird.

Auf aliphatischen Isocyanaten basierte Systeme zeigen eine nur ungenügende Reaktivität und damit eine zu langsame Aushärtungszeit. Durch den Einsatz von Metallkatalysatoren konnte, wie in EP-A 0 482 467 beschrieben, zwar die Reaktionsgeschwindigkeit erhöht werden, doch kam es zur Bildung eines Schaumes, mit den oben beschriebenen Problemen.

Eine grundsätzliche Eignung von Asparaginsäureestern zur Vernetzung von Prepolymeren ist im Stand der Technik im Rahmen von Oberflächenbeschichtungen durchaus bekannt und beispielsweise in EP-A 1 081 171 oder DE-A 102 46 708 beschrieben.

Die der vorliegenden Erfindung zugrunde liegende Aufgabe bestand nun darin, einen Gewebekleber herzustellen, der:
- eine starke Bindung zum Gewebe ausbildet
- einen transparenten Film bildet
- eine flexible Fügenaht formt
- durch eine eingestellte Viskosität leicht applizierbar ist und nicht in tiefere Gewebeschichten eindringen kann
- je nach Anwendungsgebiet eine Aushärtungszeit von wenigen Sekunden bis zu 10 Minuten hat
- keine wesentliche Exothermie beim Aushärten zeigt
- biokompatibel ist und der, sowie dessen Abbauprodukte, keine Zell- und Gewebetoxizität zeigt

Unter Gewebe im Rahmen der vorliegenden Erfindung werden Zellverbände verstanden, die aus Zellen gleicher Gestalt und Leistung bestehen wie Deckgewebe (Haut), Epithelgewebe, Myocard, Binde- oder Stützgewebe, Muskeln, Nerven und Knorpel zu verstehen. Hierzu gehören unter anderem auch alle aus Zellverbänden aufgebaute Organe wie Leber, Niere, Lunge, Herz etc.

Überraschenderweise wurde nun gefunden, dass diese Aufgabe durch eine Kombination von isocyanatfunktionellen Prepolymeren auf Basis aliphatischer Isocyanate mit aminofunktionellen Asparaginsäureestem gelöst werden konnte.

Gegenstand der vorliegenden Erfindung sind daher 2-komponentige Klebstoffsysteme umfassend
A) isocyanatfunktionelle Prepolymere erhältlich aus
   A1) aliphatischen Isocyanaten und
   A2) Polyolen mit zahlenmittleren Molekulargewichten von ≥ 400 g/mol und mittleren OH-Funktionalitäten von 2 bis 6
   und
B) aminofunktionelle Asparaginsäureester der allgemeinen Formel (I) 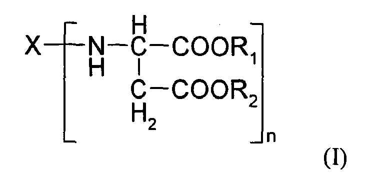 wobei
   - X: ein n-wertiger organischer Rest ist, der durch Entfernung der primären Aminogruppen eines n-wertigen Amins erhalten wird,
   - R₁, R₂: gleiche oder verschiedene organische Reste sind, die keinen Zerewitinoff-aktiven Wasserstoff aufweisen und
   - n: eine ganze Zahl von mindestens 2 ist.
   und/oder
C) Umsetzungsprodukte isocyanatfunktioneller Prepolymere mit Asparaginsäureestern gemäß Komponente B)

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Die in A) eingesetzten isocyanatfunktionellen Prepolymere sind durch Umsetzung von Isocyanaten mit hydroxyfunktionellen Polyolen gegebenenfalls unter Zusatz von Katalysatoren sowie Hilfs-und Zusatzstoffen erhältlich.

In A1) können als Isocyanate beispielsweise monomere aliphatische oder cycloaliphatische Di-oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonantriisocyanat), sowie Alkyl-2,6-diisocyanatohexanoate (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion-oder Oxadiazintrionstruktur sowie deren Mischungen eingesetzt werden.

Bevorzugt werden in A1) Isocyanate der vorstehend genannten Art mit ausschließlich aliphatisch oder cycloaliphatisch gebundenen Isocyanatgruppen oder deren Mischungen eingesetzt.

Die in A1) eingesetzten Isocyanate oder Isocyanatmischungen haben bevorzugt eine mittlere NCO-Funktionalität von 2 bis 4, besonders bevorzugt 2 bis 2,6 und ganz besonders bevorzugt 2 bis 2,4.

In einer besonders bevorzugten Ausführungsform wird in A1) Hexamethylendiisocyanat eingesetzt.

Zum Prepolymeraufbau können in A2) grundsätzliche alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolacrylatpolyole, Polyurethanpolyacrylatpolyole, Polyurethanpolyesterpolyole, Polyurethanpolyetherpolyole, Polyurethanpolycarbonatpolyole, Polyesterpolycarbonatpolyole oder deren beliebige Mischungen untereinander sein.

Die in A2) eingesetzten Polyole haben bevorzugt eine mittlere OH-Funktionalität von 3 bis 4

Die in A2) eingesetzten Polyole haben ferner bevorzugt ein zahlenmittleres Molekulargewicht von 400 bis 20000 g/mol, besonders bevorzugt 2000 bis 10000 g/mol und ganz besonders bevorzugt 4000 bis 8500.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen Gehalt an Ethylenoxid-basierten Einheiten von 50 bis 100 %, bevorzugt 60 bis 90 % bezogen auf die insgesamt enthaltene Mengen an Alkylenoxideinheiten auf.

Bevorzugte Polyesterpolyol sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt werden zum Prepolymeraufbau Polyetherpolyole der vorstehend genannten Art eingesetzt.

Zur Herstellung des Prepolymers werden die Verbindungen der Komponente A1) mit denen der Komponente A2) bei einem NCO/OH-Verhältnis von bevorzugt 4:1 bis 12:1, besonders bevorzugt 8:1 umgesetzt und anschließend der Anteil an nicht umgesetzten Verbindungen der Komponente A1) mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

Die Herstellung der aminofunktionellen Polyasparaginsäureester B) erfolgt in bekannter Weise durch Umsetzung der entsprechenden primären mindestens difunktionellen Amine X(NH₂)ₙ mit Malein- oder Fumarsäureestern der allgemeinen Formel

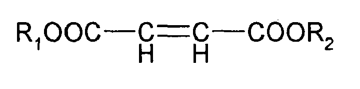

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine X(NH₂)ₙ sind Ethylendiamin, 1,2-Diaminopropan, 1,4-Diaminobutan, 1,6-Diaminohexan, 2,5-Diamino-2,5-dimethylhexan, 2,2,4-und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexylmethan, 2,4,4'-Triamino-5-methyl-dicyclohexylmethan und Polyetheramine mit aliphatisch gebundenen primären Aminogruppen mit einem zahlenmittleren Molekulargewicht Mₙ von 148 bis 6000 g/mol.

Besonders bevorzugte primäre mindestens difunktionelle Amine sind 4-Diaminobutan, 1,6-Diaminohexan, 2,2,4- und/oder 2,4,4-Trimethyl-1,6-diaminohexan.

Bevorzugt sind R₁ und R₂ unabhängig voneinander C₁ bis C₁₀-Alkylreste, besonders bevorzugt Methyl oder Ethylreste.

In einer bevorzugten Ausführungsform der Erfindung, sind R₁ = R₂ = Ethyl, wobei X auf 2,2,4-und/oder 2,4,4-Trimethyl-1,6-diaminohexan als n-wertigem Amin basiert.

Bevorzugt ist n in Formel (I) für die Beschreibung der Wertigkeit des'n-wertigen-Amins eine ganze Zahl von 2 bis 6, besonders bevorzugt 2 bis 4.

Die Herstellung der aminofunktionellen Asparaginsäureester B) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Um das Aminogruppen-Equivalentgewicht zu reduzieren ist es möglich anstatt oder zusätzlich zu den in B) eingesetzten Asparaginsäureestern auch die aminofunktionellen Umsetzungsprodukte isocyanatfunktioneller Prepolymere mit dem Asparaginsäureestern in einer separaten Vorreaktion der beiden Komponenten herzustellen und dann als höhermolekularen aminofunktonelle Härterkomponente C) einzusetzen.

Bevorzugt werden dabei Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente A) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieses Modifizierung durch Vorverlängerung der Komponente B) ist, dass das Equivalentgewicht und Equivalentvolumen der aminischen Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von Aminogruppen zu NCO-Gruppen eingestellt werden.

Die erfindungsgemäßen 2-komponentigen Klebstoffsysteme werden durch Mischung des Prepolymers mit dem aminofunktionellen Asparaginsäureestern der Komponenten B) und/oder C) erhalten. Das Verhältnis von Aminogruppen zu freien NCO-Gruppen beträgt bevorzugt 1:1,5 bis 1:1, besonders bevorzugt 1:1.

Die erfindungsgemäßen 2-komponentigen Klebstoffsysteme besitzen unmittelbar nach Vermischung der Einzelkomponenten miteinander eine Scherviskosität bei 23°C von bevorzugt 1000 bis 10000 mPas, besonders bevorzugt 2000 bis 8000 mPas und ganz besonders bevorzugt 2500 bis 5000 mPas.

Die Geschwindigkeit bei 23°C bis eine vollständige Vernetzung und Aushärtung des Klebestoffs erreicht ist, beträgt typischerweise 30 s bis 10 min, bevorzugt 1 min bis 8 min, besonders bevorzugt 1 min bis 5 min.

Ein weiterer Gegenstand der Erfindung sind die aus den erfindungsgemäßen Klebstoffsystemen erhältlichen Klebfilme sowie daraus hergestellte Verbundteile.

In einer bevorzugten Ausführungsform werden die erfindungsgemäßen 2-komponentigen Klebstoffsysteme als Gewebekleber für den Verschluss von Wunden in menschlichen oder tierischen Zellverbünden verwendet, so dass auf ein Klammern oder Nähen zum Verschließen weitestgehend verzichtet werden kann.

Der erfindungsgemäße Gewebekleber kann so wohl in vivo als auch in vitro angewendet werden, wobei die in vivo Anwendung beispielsweise zur Wundbehandlung nach Unfällen oder Operationen bevorzugt ist.

Daher ist auch ein Gegenstand der vorliegenden Erfindung ein Verfahren zum Verschließen oder Verbinden von Zellgeweben, dadurch gekennzeichnet, dass die erfindungsgemäßen 2-komponentige Klebstoffsysteme eingesetzt werden.

Ebenfalls Gegenstand der Erfindung ist ferner die Verwendung solcher 2-komponentigen Klebstoffsysteme zur Herstellung eines Mittels zum Verschließen oder Verbinden von Zellgeweben, so wie die zur Applikation notwendigen 2-Kammerdosiersysteme umfassend die erfindungswesentlichen Komponenten des Klebstoffsystems.

### Beispiele:

Sofern nicht abweichend angegeben beziehen sich alle Prozentangaben auf das Gewicht.

Als Gewebeersatz wurde Rindfleisch verwendet. Es wurden jeweils zwei Stücke Fleisch (1 = 4 cm, h = 0.3 cm, b = 1 cm) an den Enden 1 cm weit mit dem Kleber bestrichen und überlappend geklebt. Die Stabilität der Klebeschicht wurde jeweils durch Zug überprüft.

Desmophen^{®} DE 550 U: Trimethylolpropan gestartetes Propylenglycol

DE 1470 EV: N,N,N',N'-Tetrakis(2-hydroxypropyl)ethylendiamin

### Beispiel 1, (Prepolymer A-1)

465 g HDI und 2.35 g Benzoylchlorid wurden in einem 11 Vierhalskolben vorgelegt. Innerhalb von 2h wurden bei 80°C 931.8 g eines Polyethers mit einem Ethylenoxidgehalt von 63% und einem Propylenoxidgehalt von 37% (jeweils bezogen auf dem gesamten Alkylenoxidgehalt) gestartet auf TMP (3-funktionell) hinzugefügt und rührte 1h nach. Anschließend wurde durch Dünnschichtdestillation bei 130°C und 0,1 Torr das überschüssige HDI abdestilliert. Man erhielt 980 g (71 %) des Präpolymers mit einem NCO-Gehalt von 2,53%. Der Restmonomerengehalt betrug < 0,03 % HDI.

### Beispiel 2, (Prepolymer A-2)

119 g Desmodur I (IPDI) und 0.52 g Benzoylchlorid wurden in einem 1 1 Vierhalskolben vorgelegt. Innerhalb von 2h wurden bei 80°C 180.3 g eines Polyethers mit einem Ethylenoxidgehalt von 63% und einem Propylenoxidgehalt von 37% (jeweils bezogen auf dem gesamten Alkylenoxidgehalt) gestartet auf Glycerin (3-funktionell) hinzugefügt und rührte 1h nach. Anschließend wurde durch Dünnschichtdestillation bei 130°C und 0,1 Torr das überschüssige IPDI abdestilliert. Man erhielt 130 g (81 %) des Präpolymers mit einem NCO-Gehalt von 2,56%. Der Restmonomerengehalt betrug < 0,03 % HDI.

### Beispiel 3, (Aspartat B)

Zu 2 Mol Diethylmaleat wurde unter Stickstoffatmosphäre langsam 1 Mol 2,4,4-Trimethyl-1,6-diaminohexan getropft, so dass die Reaktionstemperatur 60°C nicht überschritt. Anschließend wurde so lange auf 60°C erwärmt, bis kein Diethylmaleat mehr im Reaktionsgemisch nachweisbar war.

### Beispiel 3a, (Aspartat-Komponente partiell mit Isocyanat-funktionellem Prepolymer vorverlängert)

1000 g HDI (Hexamethylendiisocyanat), 1 g Benzoylchlorid und 1 g *para-*Toluonsulfonsäuremethylester wurden in einem 4 1, Vierhalskolben unter Rühren vorgelegt. Innerhalb von 3 Stunden fügte man bei 80°C 1000 g eines difunktionellen Polypropylenglykol-Polyethers mit einem mittleren Molekulargewicht von 2000 g/mol hinzu und rührte 1h nach. Anschließend wurde durch Dünnschichtdestillation bei 130°C und 0,1 Torr das überschüssige HDI abdestilliert. Das erhaltene Prepolymer hat einen NCO-Gehalt von 3,7%.

200 g des Prepolymers wurden in einen 1l Vierhalskolben unter Rühren bei Raumtemperatur zu 291 g des Aspartats B) aus 2,4,4-Trimethyl-1,6-diaminohexan dosiert. Es wurde zwei Stunden nachgerührt, bis IR-spektroskopisch keine Isocyanatgruppen mehr nachweisbar waren. Das erhaltene Produkt hatte eine Viskosität von 3740 mPas und ein NH-Equivalentgewicht von 460 g/eq.

### Beispiel 3b, (Aspartat-Komponente partiell mit Isocyanat-funktionellem Prepolymer vorverlängert)

1000 g HDI (Hexamethylendiisocyanat), 1 g Benzoylchlorid und 1 g *para-*Toluonsulfonsäuremethylester wurden in einem 4 1 Vierhalskolben unter Rühren vorgelegt. Innerhalb von 3 Stunden fügte man bei 80°C 1000 g eines difunktionellen Polypropylenglykol-Polyethers mit einem mittleren Molekulargewicht von 8000 g/mol hinzu und rührte 1h nach. Anschließend wurde durch Dünnschichtdestillation bei 130°C und 0,1 Torr das überschüssige HDI abdestilliert. Das erhaltene Prepolymer hat einen NCO-Gehalt von 1,66%.

200 g des Prepolymers wurden 1 1 Vierhalskolben unter Rühren bei Raumtemperatur zudosiert zu 244 g des Aspartats B) aus 2,4,4-Trimethyl-1,6-diaminohexan. Es wurde zwei Stunden nachgerührt, bis IR-spektroskopisch keine Isocyanatgruppen mehr nachweisbar waren. Das erhaltene Produkt hatte eine Viskosität von 3940 mPas und ein NH-Equivalentgewicht von 460 g/eq.

### Beispiel 3c, (Aspartat-Komponente partiell mit Isocyanat-funktionellem Prepolymer A-1 vorverlängert)

200 g des Prepolymers aus A-1 wurden 1 1 Vierhalskolben unter Rühren bei Raumtemperatur zudosiert zu 200 g des Aspartats B) aus 2,4,4-Trimethyl-1,6-diaminohexan. Es wurde zwei Stunden nachgerührt, bis IR-spektroskopisch keine Isocyanatgruppen mehr nachweisbar waren. Das erhaltene Produkt hatte eine Viskosität von 11700 mPas und ein NH-Equivalentgewicht von 543 g/eq.

### Beispiel 4, Gewebekleber

10 g des Prepolymers A-1 wurden mit equivalenten Mengen des aminofunktionellen Asparaginsäureesters (Aspartat B) in einem Becher gut verrührt. Die Reaktionsmischung wurde unmittelbar danach auf das zu klebende Gewebe dünn aufgetragen.

| **Eingesetztes Amin bezogen auf Aspartat B** | **Verarbeitungszeit** | **Aushärtungszeit** **(Klebung auf Fleisch)** |
|---|---|---|
| 2,4,4-Trimethyl-1,6-diaminohexan | 10 min | 1 min |
| 4,7,10-Trioxa-1,13-tridecandiamin | 6 min | 1-5 min |
| 3,3'-Diamino-N-methyldipropylamin | 15 s | Zu schnelle Verarbeitungszeit zum Auftragen |
| 1,4-Bis-(3-aminopropyloxy)- 6 min butan | | 1-5 min |
| 1,3-Diamino-2,2-dimethylpropan | > 1 h | / |
| 1,3-Bis-(aminomethyl)-cyclohexan | 8 min | 1-5 min |
| 1,7-Diaminoheptan | 4 min | 1-5 min |
| 1,3-Diaminopropan | 3 min | 1-5 min |
| Isophorondiamin (IPDA) | > 1 h | / |
| 3a | 6 min | 1-5 min |
| 3b | 6 min | 1-5 min |
| 3c | 7 min | 1,5 min |

### Beispiel 5, Gewebekleber

10 g des Prepolymers A-2 wurden mit equivalenten Mengen des aminofunktionellen Asparaginsäureesters (Aspartat B) in einem Becher gut verrührt. Die Reaktionsmischung wurde unmittelbar danach auf das zu klebende Gewebe dünn aufgetragen. Eine Aushärtung zu einem durchsichtigen Film mit einer damit verbundenen starken Klebung hatte innerhalb von 5 min stattgefunden.

### Referenzbeispiele:

1. Bei Auftrag des Prepolymers A auf Gewebe fand keine Aushärtung und damit keine Klebung statt
2. Eine Mischung von 10 g Prepolymer A mit 1 % (0.1 g) Natriumoctoat und verschiedenen Mengen (10 bis 200 %) Wasser führte bei Auftragung auf Fleisch gemäß EP 0482467 zur Schaumbildung auf dem Gewebe. Es wurde keine Haftung beobachtet.
3. Eine Mischung von 10 g Prepolymer A mit equivalenten Mengen Triethanolamin oder DE 1470 EV wie sie häufig zur Härtung verwendet werden führte bei Auftragung auf Fleisch ebenfalls zu keiner Klebung.
4. Eine Mischung von 10 g Prepolymer A mit equivalenten Mengen an Desmophen DE 550 U als typischerweise zur Vernetzung verwendetem Polyol führte zwar vereinzelt zu einer Aushärtung auf dem Gewebe, jedoch nicht zu einer Klebung.
5. Prepolymer A wurde wie in Beispiel 1 beschrieben mit IPDI anstelle von HDI hergestellt. Das erhaltene Prepolymer wurde gemäß US 20030 135 238 mit Wassermengen von 10 % - 200 % bezogen auf das Prepolymer gemischt und auf das Gewebe aufgetragen. Es wurde keine Klebung beobachtet.
6. Prepolymer A wurde wie in Beispiel 1 beschrieben mit TDI anstelle von HDI hergestellt. Das erhaltene Prepolymer wurde gemäß US 20030135238 und US 20050129733 mit unterschiedlichen Mengen Wasser versetzt und auf Fleisch aufgetragen. Es hat eine starke Klebung unter Schaumbildung stattgefunden.

## Patentansprüche

1. 2-komponentige Klebstoffsysteme umfassend
A) isocyanatfunktionelle Prepolymere erhältlich aus
A1) aliphatischen Isocyanaten und
A2) Polyolen mit zahlenmittleren Molekulargewichten von ≥ 400 g/mol und mittleren OH-Funktionalitäten von 2 bis 6
sowie
B) aminofunktionelle Asparaginsäureester der allgemeinen Formel (I) 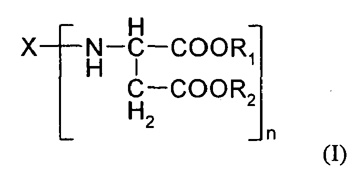 wobei
X ein n-wertiger organischer Rest ist, der durch Entfernung der primären Aminogruppen eines n-wertigen Amins erhalten wird,
R₁, R₂ gleiche oder verschiedene organische Reste sind, die keinen Zerewitinoff-aktiven Wasserstoff aufweisen und
n eine ganze Zahl von mindestens 2 ist.
und/oder
C) Umsetzungsprodukte isocyanatfunktioneller Prepolymere mit Asparaginsäureestern gemäß Komponente B).

2. 2-komponentige Klebstoffsysteme gemäß Anspruch 1, **dadurch gekennzeichnet, dass** die in A1) eingesetzten Isocyanate ausschließlich aliphatisch oder cycloaliphatisch gebundene Isocyanatgruppe aufweisen.

3. 2-komponentige Klebstoffsysteme gemäß Anspruch 1 oder 2, **dadurch gekennzeichnet, dass** die in A1) eingesetzten Isocyanate eine mittlere NCO-Funktionalität 2 bis 2,4 aufweisen.

4. 2-komponentige Klebstoffsysteme gemäß einem der Ansprüche 1 bis 3, **dadurch gekennzeichnet, dass** die in A2) eingesetzten Polyole zahlenmittlere Molekulargewichte von 4000 bis 8500 g/mol aufweisen.

5. 2-komponentige Klebstoffsysteme gemäß einem der Ansprüche 1 bis 4, **dadurch gekennzeichnet, dass** die in A2) eingesetzten Polyole mittlere OH-Funktionalitäten von 3 bis 4 aufweisen

6. 2-komponentige Klebstoffsysteme gemäß einem der Ansprüche 1 bis 5, **dadurch gekennzeichnet, dass** die in A2) Polyalkylenoxid-Polyether eingesetzt werden.

7. 2-komponentige Klebstoffsysteme gemäß Anspruch 4, **dadurch gekennzeichnet, dass** die in A2) eingesetzten Polyalkylenoxid-Polyether einen Gehalt an Ethylenoxid-basierten Einheiten von 60 bis 90 % bezogen auf die insgesamt enthaltene Mengen an Alkylenoxideinheiten aufweisen.

8. 2-komponentige Klebstoffsysteme gemäß einem der Ansprüche 1 bis 7, **dadurch gekennzeichnet, dass** die in B1) Asparaginsäureester gemäß Formel (I) eingesetzt werden, wobei
X von 4-Diaminobutan, 1,6-Diaminohexan, 2,2,4- oder 2,4,4-Trimethyl-1,6-diaminohexan als n-wertigen Aminen abgeleitet ist
R₁, R₂ unabhängig voneinander ein C₁ bis C₁₀-Alkylrest sind und
n = 2 ist.

9. 2-komponentige Klebstoffsysteme gemäß einem der Ansprüche 1 bis 8, **dadurch gekennzeichnet, dass** anstatt von Asparaginsäureestern der Komponente B) ausschließlich zur aminischen Härtung der in A) eingesetzten Prepolymere die Umsetzungsprodukte gemäß C) eingesetzt werden.

10. 2-komponentige Klebstoffsysteme gemäß einem der Ansprüche 1 bis 98, **dadurch gekennzeichnet, dass** die Umsetzungsprodukte der Komponente C) erhältlich sind durch Umsetzung der Prepolymere mit den Asparaginsäureestern in einem Verhältnis von isocyanatreaktiven Gruppen zu Isocyanatgruppen von 15 zu 1 bis 4 zu 1.

11. 2-komponentige Klebstoffsysteme gemäß einem der Ansprüche 1 bis 10, **dadurch gekennzeichnet, dass** es sich um einen Gewebekleber für menschliches oder tierisches Gewebe handelt.

12. Verfahren zum Verschließen oder Verbinden von Zellgeweben, **dadurch gekennzeichnet, dass** 2-komponentige Klebstoffsysteme gemäß einem der Ansprüche 1 bis 11 eingesetzt werden.

13. Verfahren gemäß Anspruch 12, **dadurch gekennzeichnet, dass** es sich bei dem Zellgewebe um menschliches oder tierisches Gewebe handelt.

14. Verwendung 2-komponentige Klebstoffsysteme gemäß einem der Ansprüche 1 bis 11 zur Herstellung eines Mittels zum Verschließen oder Verbinden von Zellgeweben.

15. Klebstofffilme und Verbundteile erhältlich unter Verwendung von 2-komponentigen Klebstoffsystemen gemäß einem der Ansprüche 1 bis 9.

16. 2-Kammerdosiersystem umfassend ein 2-komponentiges Klebstoffsystem gemäß einem der Ansprüche 1 bis 11.
